(19) Europäisches Patentamt
European Patent Office
Office européen des brevets

(11) EP 4 269 423 A1

(12) **EUROPEAN PATENT APPLICATION**
published in accordance with Art. 153(4) EPC

(43) Date of publication:
**01.11.2023 Bulletin 2023/44**

(21) Application number: **21910748.9**

(22) Date of filing: **21.12.2021**

(51) International Patent Classification (IPC):
***C07K 1/06*** (2006.01) ***C07C 269/06*** (2006.01)
***C07C 271/22*** (2006.01) ***C07D 311/88*** (2006.01)

(52) Cooperative Patent Classification (CPC):
**C07C 269/06; C07C 271/22; C07D 311/88; C07K 1/06;** Y02P 20/55

(86) International application number:
**PCT/JP2021/047183**

(87) International publication number:
**WO 2022/138605 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.12.2020 JP 2020212819**

(71) Applicant: **FUJIFILM Corporation**
**Tokyo 106-8620 (JP)**

(72) Inventor: **TSUYAMA, Hiroaki**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

# (54) METHOD FOR PRODUCING PEPTIDE, REAGENT FOR FORMING PROTECTIVE GROUP, AND FUSED POLYCYCLIC COMPOUND

(57) An object of the present invention is to provide a method for producing a peptide with high purity and high efficiency, a protective group-forming reagent having excellent deprotective properties, and a novel condensed polycyclic compound. According to the present invention, there are provided a method for producing a peptide including a step of using a polycyclic compound represented by Formula (1), a protective group-forming reagent containing the polycyclic compound, and the polycyclic compound. At least one of $R^1$ to $R^8$ and $R^{110}$ has $R^A$, $R^A$ represents an aliphatic hydrocarbon group or an organic group having an aliphatic hydrocarbon group, and at least one aliphatic hydrocarbon group has 3 or more carbon atoms.

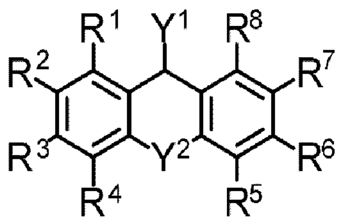
(1)

## Description

Technical Field

The present disclosure relates to a method for producing a peptide, a protective group-forming reagent, and a condensed polycyclic compound.

Background Art

Examples of a method for producing peptide include a solid phase method and a liquid phase method.

The liquid phase method is advantageous in that good reactivity is exhibited, and intermediate peptide can be purified by extraction and washing, isolation, and the like after a condensation reaction. However, depending on the peptide to be synthesized, the performance of an existing side chain protective group may be insufficient.

As a protective group-forming reagent in the related art, a xanthene compound disclosed in Patent Document 1 and a diphenylmethane compound disclosed in Patent Document 2 have been known.

Prior Art Documents

Patent Documents

Patent Document 1: WO2018/021233A
Patent Document 2: WO2010/113939A

## SUMMARY OF THE INVENTION

Patent Document 1 only discloses a xanthene compound which is difficult to precipitate in an organic solvent and can be easily separated and purified by a liquid-liquid phase separation operation.

An object to be achieved by an embodiment of the present disclosure is to provide a method for producing a peptide with high purity and high efficiency.

An object to be achieved by another embodiment of the present disclosure is to provide a protective group-forming reagent having excellent deprotective properties.

An object to be achieved by still another embodiment of the present disclosure is to provide a novel condensed polycyclic compound.

The methods for achieving the above-described objects include the following aspects.

< 1 > A method for producing a peptide, comprising:

a step of using a polycyclic compound represented by Formula (1),

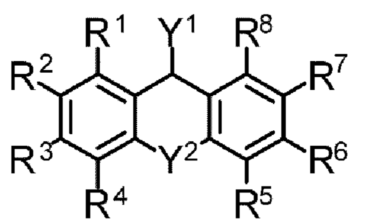

(1)

in Formula (1), $Y^1$ represents $-OR^{17}$, $-NHR^{18}$, $-NHCOR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group, an arylalkyl group, a heteroarylalkyl group, or a chemical structure having a protected or unprotected amino group and a carboxy group,

$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$ each independently represent a hydrogen atom or an alkyl group, and $R^{110}$ represents $R^A$ or a hydrogen atom,

$R^1$ to $R^8$ each independently represent $R^A$, a hydrogen atom, a halogen atom, an alkyl group, or an alkoxy group, where at least one of $R^1$ to $R^8$ and $R^{110}$ is $R^A$, and

$R^A$'s each independently represent an aliphatic hydrocarbon group or an organic group having an aliphatic

hydrocarbon group, where at least one aliphatic hydrocarbon group has 3 or more carbon atoms.

<2> The method for producing a peptide according to <1>,
in which the step of using the compound represented by Formula (1) is a side chain protecting step of protecting a protected or unprotected side chain amino group of asparagine or glutamine with the compound represented by Formula (1).
<3> The method for producing a peptide according to < 1>,

in which the compound represented by Formula (1) is an N-terminal protected amino acid or an N-terminal protected peptide, and
the method for producing a peptide further comprising:

a peptide chain extending step of condensing the compound with a C-terminal protected amino acid or a C-terminal protected peptide; and
a precipitating step of precipitating an N-terminal and C-terminal protected peptide obtained in the peptide chain extending step.

<4> The method for producing a peptide according to <3>, further comprising, one or more times in the following order after the precipitating step:

a step of deprotecting an N-terminal of the obtained N-terminal and C-terminal protected peptide;
a step of condensing an N-terminal of the obtained C-terminal protected peptide with an N-terminal protected amino acid or an N-terminal protected peptide; and
a step of precipitating the obtained N-terminal and C-terminal protected peptide.

<5> The method for producing a peptide according to <3> or <4>, further comprising:

a C-terminal deprotecting step of deprotecting a C-terminal protective group,
in which the deprotecting is performed using a trifluoroacetic acid solution of 10% by volume or less.

<6> The method for producing a peptide according to any one of <3> to <5>,
in which a C-terminal protective group of the C-terminal protected amino acid or the C-terminal protected peptide has an aliphatic hydrocarbon group having 12 or more carbon atoms.
<7> The method for producing a peptide according to any one of <1> to <6>,
in which only one of $R^1$ to $R^8$ in Formula (1) is $R^A$.
<8> The method for producing a peptide according to any one of <1> to <7>,
in which $R^3$ or $R^6$ in Formula (1) is $R^A$.
<9> The method for producing a peptide according to any one of <1> to <8>,
in which $R^A$'s in Formula (1) are each independently an alkyl group having 3 to 10 carbon atoms, an alkoxy group having 3 to 10 carbon atoms, or an alkoxyalkyl group having 3 to 10 carbon atoms.
<10> The method for producing a peptide according to any one of <1> to <7>,
in which $Y^2$ in Formula (1) is -O-.
<11> A protective group-forming reagent of a side chain amino of arginine or glutamine, comprising:

a polycyclic compound represented by Formula (1),

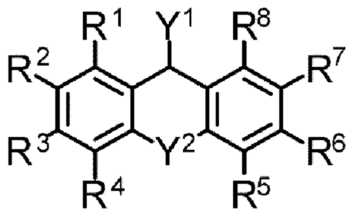
(1)

in Formula (1), $Y^1$ represents $-OR^{17}$, $-NHR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group, an arylalkyl group, or a heteroarylalkyl group,
$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$

each independently represent a hydrogen atom or an alkyl group, and $R^{110}$ represents $R^A$ or a hydrogen atom, $R^1$ to $R^8$ each independently represent $R^A$, a hydrogen atom, a halogen atom, an alkyl group, or an alkoxy group, where at least one of $R^1$ to $R^8$ and $R^{110}$ is $R^A$, and
$R^A$ represents an aliphatic hydrocarbon group or an organic group having an aliphatic hydrocarbon group, where at least one aliphatic hydrocarbon group has 3 to 10 carbon atoms.

< 12> A polycyclic compound represented by Formula (1),

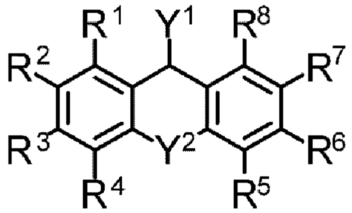
(1)

in Formula (1), $Y^1$ represents $-OR^{17}$, $-NHR^{18}$, $-NHCOR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group having 10 or less carbon atoms, an arylalkyl group, a heteroarylalkyl group, or a chemical structure having a protected or unprotected amino group and a carboxy group,
$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$ each independently represent a hydrogen atom or an alkyl group, and $R^{110}$ represents $R^A$ or a hydrogen atom,
$R^1$ to $R^8$ each independently represent $R^A$, a hydrogen atom, a halogen atom, an alkyl group having 1 to 4 carbon atoms, or an alkoxy group having 1 to 4 carbon atoms,
at least one of $R^2$ to $R^7$ and $R^{110}$ is $R^A$, and
$R^A$ represents an aliphatic hydrocarbon group or an organic group having an aliphatic hydrocarbon group, where at least one aliphatic hydrocarbon group has 3 to 10 carbon atoms.

<13> The polycyclic compound according to claim 12,

in which the polycyclic compound is represented by Formula (2),

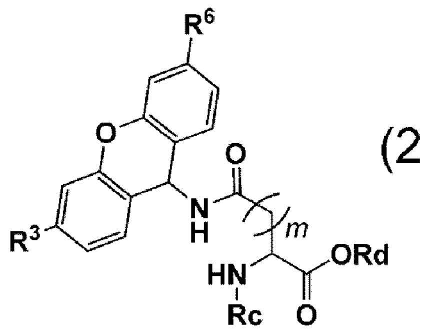
(2)

in Formula (2), $R^3$ and $R^6$ each independently represent a hydrogen atom or an alkoxy group having 3 to 10 carbon atoms, where at least one of $R^3$ or $R^6$ is an alkoxy group having 2 to 10 carbon atoms,
Rc represents a hydrogen atom, a tert-butoxycarbonyl group, or a 9-fluorenylmethoxycarbonyl group, Rd represents a hydrogen atom or a cation having a salt structure, and m represents 1 or 2.

According to an embodiment of the present invention, it is possible to provide a method for producing a peptide with high purity and high efficiency.
In addition, according to another embodiment of the present invention, it is possible to provide a protective group-forming reagent having excellent deprotective properties.
In addition, according to still another embodiment of the present invention, it is possible to provide a novel condensed polycyclic compound.

## DESCRIPTION OF THE PREFERRED EMBODIMENTS

Hereinafter, the content of the present disclosure will be described in detail. The description of configuration requirements below is made based on representative embodiments of the present disclosure in some cases, but the present disclosure is not limited to such embodiments.

In the present specification, unless otherwise specified, each term has the following meaning.

A numerical range represented using "to" means a range including numerical values described before and after the preposition "to" as a lower limit value and an upper limit value.

In numerical ranges described in stages in the present specification, an upper limit value or a lower limit value described in one numerical range may be replaced with an upper limit value or a lower limit value of a numerical range described in another stage. In addition, in the numerical ranges described in the present specification, the upper limit value or the lower limit value of the numerical ranges may be replaced with the values shown in examples.

The term "step" includes not only the independent step but also a step in which intended purposes are achieved even in a case where the step cannot be precisely distinguished from other steps.

In a case where substitution or unsubstitution is not noted in regard to the notation of a "group" (atomic group), the "group" includes not only a group not having a substituent but also a group having a substituent. For example, the concept of an "alkyl group" includes not only an alkyl group not having a substituent (unsubstituted alkyl group) but also an alkyl group having a substituent (substituted alkyl group).

A chemical structural formula may be described by a simplified structural formula in which hydrogen atoms are omitted.

"% by mass" has the same definition as that for "% by weight", and "part by mass" has the same definition as that for "part by weight".

A combination of two or more preferred aspects is a more preferred aspect.

"Alkyl group" may be chain-like or branched, and may be substituted with a halogen atom or the like.

The "alkyl group" is preferably an alkyl group having 1 to 10 carbon atoms (also referred to as "the number of carbon atoms"). The number of carbon atoms in the alkyl group is more preferably 1 to 6, still more preferably 1 to 4, and even more preferably 1 or 2. Specific examples thereof include methyl, ethyl, propyl, isopropyl, butyl, and tert-butyl.

"Alkenyl group" is preferably an alkenyl group having 2 to 6 carbon atoms. Specific examples thereof include 1-propenyl.

As "aryl group", an aryl group having 6 to 14 carbon atoms is preferable, and examples thereof include a phenyl group, a 1-naphthyl group, a 2-naphthyl group, a biphenylyl group, and a 2-anthryl group. Among these, an aryl group having 6 to 10 carbon atoms is more preferable, and a phenyl group is particularly preferable.

Examples of "silyl group" include trimethylsilyl, triethylsilyl, dimethylphenylsilyl, tert-butyldimethylsilyl, and tert-butyldiethylsilyl.

Examples of "halogen atom" include a fluorine atom, a chlorine atom, a bromine atom, and an iodine atom.

As "alkoxy group", an alkoxy group having 1 to 10 carbon atoms is preferable. The number of carbon atoms in the alkoxy group is more preferably 1 to 6, still more preferably 1 to 4, and even more preferably 1 or 2. Specific examples thereof include methoxy, ethoxy, and propoxy.

As "arylalkyl group", an arylalkyl group having 7 to 20 carbon atoms is preferable. The number of carbon atoms is preferably 7 to 12. Specific examples thereof include benzyl.

As "heteroarylalkyl group", a heteroarylalkyl group having 6 to 20 carbon atoms is preferable. The number of carbon atoms is preferably 6 to 12.

As "acyl group", an acyl group having 1 to 6 carbon atoms is preferable. Specific examples thereof include acetyl and propionyl.

As "arylalkylcarbonyl group", an arylalkylcarbonyl having 7 to 10 carbon atoms is preferable. Specific examples thereof include benzylcarbonyl.

As "alkoxycarbonyl group", an alkoxycarbonyl group having 1 to 6 carbon atoms is preferable. Specific examples thereof include methoxycarbonyl, ethoxycarbonyl, and a Boc group. The Boc group means a tert-butoxycarbonyl group.

As "arylalkyloxycarbonyl group", an arylalkyloxycarbonyl group having 7 to 20 carbon atoms is preferable. The number of carbon atoms is preferably 8 to 20 and more preferably 8 to 14. Specific examples thereof include benzyloxycarbonyl (hereinafter, also referred to as a Cbz group or a Z group) and an Fmoc group. The Fmoc group means a 9-fluorenylmethoxycarbonyl group.

In a case where the amino acid and peptide according to the present disclosure have a hydroxy group, an amino group, a carboxy group, a carbonyl group, an amide group, a guanidyl group, a mercapto group, or the like, these groups may be protected, and a target compound can be obtained by removing a protective group after the reaction, as necessary.

Examples of a protective group of the hydroxy group include an alkyl group, an aryl group, a trityl group, an arylalkyl group having 7 to 10 carbon atoms, a formyl group, an acyl group having 1 to 6 carbon atoms, a benzoyl group,

an arylalkylcarbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with one to three substituents selected from the group consisting of a halogen atom, an alkyl group having 1 to 6 carbon atoms, an alkoxy group having 1 to 6 carbon atoms, and a nitro group.

Examples of a protective group of the amino group include a formyl group, an acyl group having 1 to 6 carbon atoms, an alkoxycarbonyl group having 1 to 6 carbon atoms, a benzoyl group, an arylalkylcarbonyl group having 7 to 10 carbon atoms, an arylalkyloxycarbonyl group having 7 to 14 carbon atoms, a trityl group, a monomethoxytrityl group, a 1-(4,4-dimethyl-2,6-dioxocyclohex-1-ylidene)-3-methylbutyl group, a phtaloyl group, an N,N-dimethylaminomethylene group, a silyl group, and an alkenyl group having 2 to 6 carbon atoms. These groups may be substituted with one to three substituents selected from the group consisting of a halogen atom, an alkoxy group having 1 to 6 carbon atoms, and a nitro group.

Examples of a protective group of the carboxy group include the above-described protective group of the hydroxy group and a trityl group.

Examples of a protective group of the amide group include a trityl group.

Examples of a protective group of the carbonyl group include cyclic acetals (for example, 1,3-dioxane) and acyclic acetals (for example, di(alkyl having 1 to 6 carbon atoms) acetal).

Examples of a protective group of the guanidyl group include a 2,2,4,6,7-pentamethyldihydrobenzofuran-5-sulfonyl group, a 2,3,4,5,6-pentamethylbenzenesulfonyl group, a tosyl group, and a nitro group.

Examples of a protective group of the mercapto group (thiol group) include a trityl group, a 4-methylbenzyl group, an acetylaminomethyl group, a t-butyl group, and a t-butylthio group.

The method of removing the above-described protective group may be performed according to a known method described in, for example, Protective Groups in Organic Synthesis, John Wiley and Sons (1980). A method using acid, base, ultraviolet light, hydrazine, phenylhydrazine, sodium N-methyldithiocarbamate, tetrabutylammonium fluoride, palladium acetate, or trialkylsilyl halide, or a reduction method is used.

"Amino acid" is an $\alpha$, $\beta$, or $\gamma$ amino acid, and is not limited to a naturally occurring amino acid and may be a non-naturally occurring amino acid. In addition, the "Amino acid" may be an amino acid analog such as hydroxycarboxylic acid.

(Polycyclic compound represented by Formula (1))

The polycyclic compound according to the embodiment of the present disclosure is represented by Formula (1), and is preferably a compound represented by Formula (2). Such a compound is the polycyclic compound and protective group-forming reagent according to the embodiment of the present invention, and is used in the method for producing a peptide according to the embodiment of the present invention.

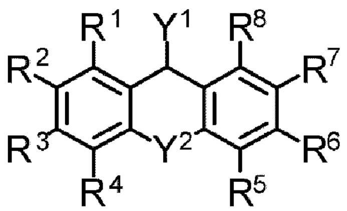
(1)

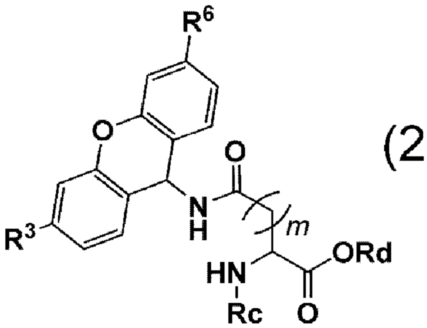
(2)

In Formula (1), $Y^1$ represents $-OR^{17}$, $-NHR^{18}$, $-NHCOR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group, an arylalkyl group, a heteroarylalkyl group, or a chemical structure having a protected or unprotected

amino group and a carboxy group. As the halogen atom, from the viewpoint of reaction yield and storage stability, a bromine atom or a chlorine atom is preferable.

Examples of the active ester-type carbonyl group in $R^{17}$ include carbonyloxysuccinate imide, an alkoxycarbonyl group, an aryloxycarbonyl group, and an arylalkyloxycarbonyl group, and from the viewpoint of deprotective properties, carbonyloxysuccinate imide is preferable.

Examples of the active ester-type sulfonyl group in $R^{17}$ include an alkylsulfonyl group and an arylsulfonyl group, and from the viewpoint of deprotective properties, an alkylsulfonyl group having 1 to 6 carbon atoms or a p-toluenesulfonyl group is preferable.

$R^{17}$ is preferably a hydrogen atom or an active ester-type protective group, and more preferably a hydrogen atom.

As the arylalkyl group in $R^{18}$, an arylalkyl group having 7 to 20 carbon atoms is preferable, and an arylalkyl group having 7 to 16 carbon atoms is more preferable.

As the heteroarylalkyl group in $R^{18}$, a heteroarylalkyl group having 5 to 30 carbon atoms is preferable, and a heteroarylalkyl group having 5 to 20 carbon atoms is more preferable.

As the chemical structure having a protected or unprotected amino group and a carboxy group in $R^{18}$, a chemical structure represented by Formula (2a) is preferable.

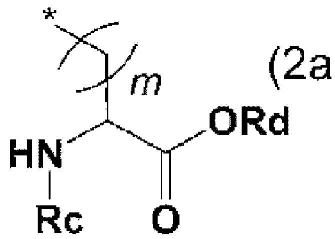

(2a)

In Formula (2a), Rc represents a hydrogen atom, a tert-butoxycarbonyl group, or a 9-fluorenylmethoxycarbonyl group, Rd represents a hydrogen atom or a cation having a salt structure, m represents 1 or 2, and * represents a bonding site.

$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{110}$ represents $R^A$ or a hydrogen atom and $R^{100}$ to $R^{107}$ each independently represent a hydrogen atom or an alkyl group.

$R^{110}$ is preferably $R^A$. $R^A$ has the meaning as $R^A$ which will be described later, and the preferred aspects thereof are also the same.

As the alkyl group in $R^{100}$ to $R^{107}$, an alkyl group having 1 to 6 carbon atoms is preferable, and an alkyl group having 1 to 3 carbon atoms is more preferable.

It is preferable that each of $R^{100}$ and $R^{101}$ in $-CR^{100}=CR^{101}-$ is the same group, and an alkyl group having 1 to 6 carbon atoms or a hydrogen atom is more preferable. Specific examples thereof include -CH=CH- and $-C(CH_3)=C(CH_3)-$.

As $R^{102}$ to $R^{105}$ in $-CR^{102}R^{103}-CR^{104}R^{105}-$, it is preferable that $R^{102}$ and $R^{104}$, and $R^{103}$ and $R^{105}$ are the same groups, respectively.

As $R^{102}$ and $R^{104}$, an alkyl group having 1 to 6 carbon atoms or a hydrogen atom is preferable. As $R^{103}$ and $R^{105}$, an alkyl group having 1 to 6 carbon atoms or a hydrogen atom is preferable. Specific examples thereof include $-CH_2-CH_2-$.

It is preferable that each of $R^{106}$ and $R^{107}$ in $-CR^{106}R^{107}-$ is the same group, and an alkyl group having 1 to 6 carbon atoms is more preferable. Specific examples thereof include $-CH_2-$, $-C(CH_3)_2-$, and $-C(C_2H_5)_2-$.

As $Y^2$, an oxygen atom (-O-) or a sulfur atom (-S-) is preferable, and an oxygen atom (-O-) is more preferable.

$R^1$ to $R^8$ each independently represent $R^A$, a hydrogen atom, a halogen atom, an alkyl group (preferably an alkyl group halogen 1 to 10 carbon atoms), or an alkoxy group (preferably an alkoxy group having 1 to 10 carbon atoms), in which at least one of $R^1$ to $R^8$ and $R^{110}$ is $R^A$.

In a case where the polycyclic compound represented by Formula (1) is used in the method for producing a peptide, $R^A$ represents "aliphatic hydrocarbon group" or "organic group having an aliphatic hydrocarbon group", and in a case where only one $R^A$ is present, the number of carbon atoms in the aliphatic hydrocarbon group of $R^A$ is 3 or more, and in a case where a plurality of $R^A$'s are present, the number of carbon atoms in at least one of the aliphatic hydrocarbon group of $R^A$ is 3 or more.

However, $R^A$ does not have a silyl group and a hydrocarbon group having a silyloxy structure.

In the protective group-forming reagent of a side chain amino of arginine or glutamine according to the embodiment of the present disclosure and the compound according to the embodiment of the present disclosure, the number of carbon atoms in at least one aliphatic hydrocarbon group of $R^A$ is 3 to 10.

It is preferable that only one of $R^1$ to $R^8$ is $R^A$, and it is more preferable that only $R^3$ or $R^6$ is $R^A$. In this case,

the substituent other than $R^A$ is preferably a hydrogen atom or a halogen atom, and more preferably a hydrogen atom.

The "aliphatic hydrocarbon group" in $R^A$ is a linear, branched, or cyclic saturated or unsaturated aliphatic hydrocarbon group. The number of carbon atoms in the aliphatic hydrocarbon group is more preferably 3 to 10, still more preferably 3 to 8, and even more preferably 4 to 8. Examples of the aliphatic hydrocarbon group include an alkyl group, a cycloalkyl group, an alkenyl group, and an alkynyl group, and an alkyl group is preferable.

The "organic group having an aliphatic hydrocarbon group" in $R^A$ is a monovalent (one bonding site bonded to the condensed polycycle) organic group having an aliphatic hydrocarbon group in its molecular structure.

The moiety of the "aliphatic hydrocarbon group" in the "organic group having an aliphatic hydrocarbon group" is not particularly limited, and may be present at the terminal (a monovalent group) or may be present at any other site (for example, a divalent group).

A moiety other than the "aliphatic hydrocarbon group" in the "organic group having an aliphatic hydrocarbon group" can be optionally set. For example, the "organic group having an aliphatic hydrocarbon group" may have a moiety such as -O-, -S-, -COO-, -OCONH-, -CONH-, and a hydrocarbon group (monovalent group or divalent group) other than the "aliphatic hydrocarbon group".

The bond between the "organic group having an aliphatic hydrocarbon group" and the condensed polycycle may be through "aliphatic hydrocarbon group" or "hydrocarbon group", that is, the "organic group having an aliphatic hydrocarbon group" and the condensed polycycle may be bonded through a direct carbon-carbon bond or may be bonded through a moiety other than "aliphatic hydrocarbon group".

From the viewpoint of ease of synthesis, it is preferable that the bond (substitution) of the "organic group having an aliphatic hydrocarbon group" to N in $Y^2$ is through the "hydrocarbon group" existing in $R^A$ described above, that is, directly bonded by a carbon-nitrogen bond.

As $R^A$, an alkyl group having 3 to 10 carbon atoms, an alkoxy group having 3 to 10 carbon atoms, or an alkoxyalkyl group having 3 to 10 carbon atoms is preferable, and an alkoxy group having 3 to 10 carbon atoms is more preferable. The number of carbon atoms in these substituents is more preferably 3 to 8, still more preferably 4 to 8, even more preferably 5 to 8, and particularly preferably 6.

According to one embodiment of the present disclosure, by using a protective group having excellent deprotective properties, it is possible to provide a method for producing a peptide with high purity and high synthesis efficiency. Here, "excellent deprotective properties" means that a deprotection rate with a weak acid is high. The weak acid means an acidic solution of trifluoroacetic acid of 20% by volume or less, and the concentration of trifluoroacetic acid is preferably 10% by volume or less, more preferably 5% by volume or less, still more preferably 2% by volume or less, and even more preferably 1% by volume or less. The lower limit thereof is preferably 0.01% by volume and more preferably 0.1% by volume.

Furthermore, even poorly synthesized peptides such as unnatural peptide including unnatural amino acid, in which a side reaction is likely to occur, the peptide can be synthesized with high purity due to suppression of the side reaction.

In the production method of the present disclosure, it is possible to deprotect the side chain protective group even under weak acid conditions, and it is possible to suppress a side reaction of the obtained peptide, which is suitable for the synthesis of peptide containing asparagine and/or glutamine.

From the viewpoint of deprotective properties, the polycyclic compound represented by Formula (1) is preferably a compound represented by Formula (10).

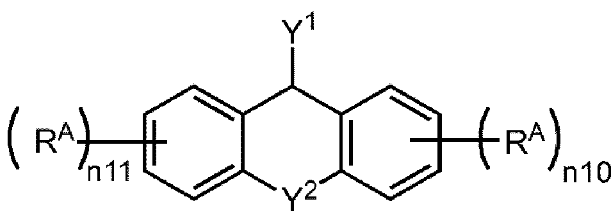

(10)

$Y^1$, $Y^2$, and $R^A$ in Formula (10) have the same meaning as $Y^1$, $Y^2$, and $R^A$ in Formula (1), respectively, and the preferred aspects thereof are also the same. n10 and n11 each independently represent an integer of 0 to 4, in which both n10 and n11 are not 0.

n10 and n11 are each independently preferably an integer of 0 to 2, and it is more preferable that any one of n10 or n11 is 0 and the other is 1.

It is preferable that $R^A$ is bonded to any of a 2-position, a 3-position, a 4-position, a 5-position, a 6-position, or a 7-position of the condensed polycycle, it is more preferable to be bonded to any of a 2-position, a 3-position, a 6-position, or a 7-position of the condensed polycycle, and it is still more preferable to be bonded to a 3-position or a 6-position of the condensed polycycle.

The compound represented by Formula (10) is preferably a compound represented by Formula (100) or Formula (200), and more preferably a compound represented by Formula (100).

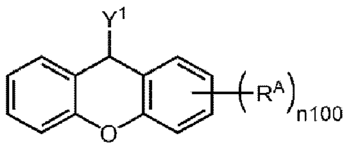 (100) 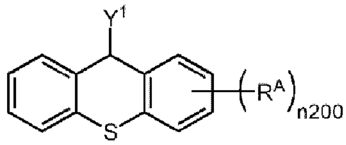 (200)

$Y^1$ and $R^A$ in Formula (100) or Formula (200) have the same meaning as $Y^1$ and $R^A$ in Formula (1), respectively, and the preferred aspects thereof are also the same. n100 and n200 each independently represent an integer of 1 to 4, preferably an integer of 1 to 3, more preferably 1 or 2, and still more preferably 1.

It is preferable that $R^A$ is bonded to any of a 2-position, a 3-position, or a 4-position of the condensed polycycle, it is more preferable to be bonded to any of a 2-position or a 3-position of the condensed polycycle, and it is still more preferable to be bonded to a 3-position of the condensed polycycle.

A molecular weight of the polycyclic compound represented by Formula (1) is not particularly limited, but from the viewpoint of deprotection rate, crystallization property, solubility in a solvent, and yield, it is preferably 340 to 3,000, more preferably 400 to 2,000, and particularly preferably 500 to 1,300.

<Method for producing polycyclic compound represented by Formula (1)>

The method for producing the polycyclic compound represented by Formula (1) according to the present disclosure is not particularly limited, and the polycyclic compound represented by Formula (1) can be produced by referring to a known method.

Unless otherwise specified, a raw material compound used for producing the polycyclic compound represented by Formula (1) may be a commercially available compound, or may be produced by a known method or a method according to the known method.

In addition, the produced polycyclic compound represented by Formula (1) may be purified by a known purification method as necessary. For example, a method of isolating and purifying by recrystallization, column chromatography, or the like, a method of purifying by reprecipitation with a unit for changing the solution temperature, a unit for changing the solution composition, or the like, and the like can be performed.

The method for synthesizing the polycyclic compound represented by Formula (1) according to the present disclosure is not particularly limited, but the polycyclic compound represented by Formula (1) can be synthesized according to, for example, the following scheme using 3-hydroxyxanthone and the like as a starting material. In addition, it is also possible to synthesize by referring to the synthesis method described in WO2018/021233A.

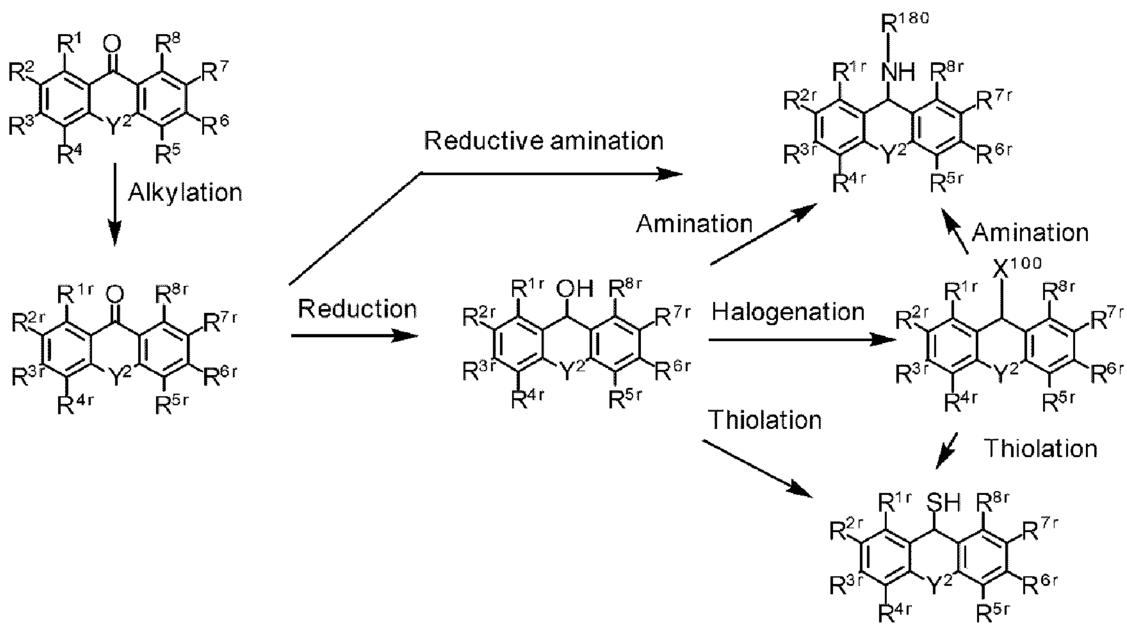


$R^{1r}$ to $R^{8r}$ each independently represent $R^A$, a hydrogen atom, a halogen atom, an alkyl group, or an alkoxy

group, where at least one of $R^{1r}$ to $R^{8r}$ and $R^{110}$ represents $R^A$. $X^{100}$ represents Cl, Br, or I. $R^{180}$ represents a hydrogen atom, an alkyl group, or an Fmoc group.

(Method for producing peptide)

In the method for producing a peptide according to the embodiment of the present disclosure, it is preferable that the step of using the above-described polycyclic compound represented by Formula (1) is a side chain protecting step of protecting a side chain of an amino acid or a peptide with the above-described polycyclic compound represented by Formula (1).

In the method for producing a peptide according to the embodiment of the present disclosure, the polycyclic compound represented by Formula (1) can be used not only for formation of a protective group, but also for denaturation of a peptide, adjustment of solubility in water or an organic solvent, improvement of crystallinity, multimerization, and the like.

Among these, the polycyclic compound represented by Formula (1) is preferably used for forming a protective group, and more preferably used as a protective group for an amide group which is a side chain of asparagine and glutamine.

From the viewpoint of ease of synthesis of the peptide and yield, it is more preferable that the method for producing a peptide according to the embodiment of the present disclosure further includes, in addition to the above-described side chain protecting step, an N-terminal deprotecting step of deprotecting an N-terminal of the N-terminal and C-terminal protected amino acid or N-terminal and C-terminal protected peptide obtained in the above-described side chain protecting step, and a peptide chain extending step of condensing an N-terminal protected amino acid or an N-terminal protected peptide to the N-terminal of the C-terminal protected amino acid or C-terminal protected peptide obtained in the above-described N-terminal deprotecting step;

it is still more preferable to further include a precipitating step of precipitating the N-terminal and C-terminal protected peptide obtained in the above-described peptide chain extending step; and
it is even more preferable to further include, one or more times in the following order, a step of, after the above-described precipitating step, deprotecting an N-terminal of the obtained N-terminal and C-terminal protected peptide, a step of condensing an N-terminal protected amino acid or an N-terminal protected peptide to the N-terminal of the obtained C-terminal protected peptide, and a step of precipitating the obtained N-terminal and C-terminal protected peptide.

The N-terminal protected amino acid or the N-terminal protected peptide is an amino acid or a peptide in which only the N-terminal is protected, and the N-terminal and C-terminal protected amino acid or peptide is an amino acid or a peptide in which the N-terminal and the C-terminal are protected.

The formation reaction (condensation reaction) of the peptide bond between the amino group and the carboxy group and the deprotection of the protective group in each step can be carried out by a known method. For example, WO2020/175473A and WO2020/175472A are referred to, which are incorporated herein by reference.

Hereinafter, each step described above will be described in detail.

<Dissolving step>

The method for producing a peptide according to the embodiment of the present disclosure preferably includes, before the above-described side chain protecting step or peptide chain extending step, a dissolving step of dissolving the polycyclic compound represented by Formula (1) in a solvent.

As the solvent, a general organic solvent can be used for the reaction, but since excellent reactivity can be expected as solubility in the above-described solvent is higher, a solvent having a high solubility of the polycyclic compound represented by Formula (1) is selected. Specific examples thereof include halogenated hydrocarbons such as chloroform and dichloromethane; and nonpolar organic solvents such as 1,4-dioxane, tetrahydrofuran (THF), and cyclopentyl methyl ether. Two or more kinds of these solvents may be mixed and used. In addition, as long as the polycyclic compound represented by Formula (1) can be dissolved, in the above-described halogenated hydrocarbons or nonpolar organic solvents, aromatic hydrocarbons such as benzene, toluene, and xylene; nitriles such as acetonitrile and propionitrile; ketones such as acetone and 2-butanone; amides such as N,N-dimethylformamide and N-methylpyrrolidone; and sulfoxides such as dimethyl sulfoxide may be mixed and used.

As the solvent, a non-polar organic solvent is preferable, and THF is more preferable. According to the present invention, even in a case where the non-polar organic solvent is used, high reactivity can be maintained and a production yield can be increased.

In addition, a solvent described in Organic Process Research & Development, 2017, 21, 3, 365 to 369 may be

used.

<Side chain protecting step>

The method for producing a peptide according to the embodiment of the present disclosure preferably includes a side chain protecting step of protecting a side chain amino group of an amino acid or a peptide with the above-described polycyclic compound represented by Formula (1). The protected amino acid is preferably asparagine or glutamine, and these may be protected from other amino groups or carboxy groups. In addition, in a case where the side chain of the peptide is protected, it is preferable that a side chain of a portion derived from asparagine or glutamine is protected. The asparagine or glutamine has lower reaction rate, a lower deprotection ratio, and a lower production efficiency, but by using the compound according to the embodiment of the present invention, such problems are improved.
In this case, in the polycyclic compound represented by Formula (1), it is preferable that $Y^1$ is $-OR^{17}$.
In a case of using the above-described polycyclic compound represented by Formula (1) in which $Y^1$ in Formula (1) is -OH, it is preferable to carry out the reaction in a solvent which does not affect the reaction in the presence of an acid catalyst. As the acid catalyst, WO2020/175473A and WO2020/175472A are referred to, which are incorporated herein by reference.
As the acid catalyst, an acid catalyst generally used in peptide synthesis can be used without limitation, and examples thereof include methanesulfonic acid, trifluoromethanesulfonic acid, p-toluenesulfonic acid, trifluoroacetic acid, and acetic acid.
Among these, trifluoroacetic acid or acetic acid is preferable.
An amount of the acid catalyst to be used is preferably more than 0 molar equivalent and 20 molar equivalent or less, more preferably 0.5 molar equivalent to 15 molar equivalent, and still more preferably 1 molar equivalent to 10 molar equivalent with respect to 1 molar equivalent of the above-described polycyclic compound represented by Formula (1).

<C-terminal protecting step>

The method for producing a peptide according to the embodiment of the present disclosure preferably includes a C-terminal protecting step of protecting a carboxy group or an amide group of the amino acid or the peptide with a C-terminal protective group.
As the C-terminal protective group, a compound having an aliphatic hydrocarbon group having 12 or more carbon atoms is preferable, and the number of carbon atoms is preferably 15 or more and more preferably 20 to 30. In a case where the C-terminal protective group has a plurality of aliphatic hydrocarbon groups, the total number of carbon atoms therein is preferably 30 to 80 and more preferably 36 to 80. The C-terminal protective group preferably has a ring structure, and more preferably has a condensed polycycle, an aromatic heterocyclic ring, or a naphthalene ring.
As the C-terminal protective group, an aromatic heterocyclic compound represented by Formula (1) in WO2020/175473A is preferable. For such a compound, WO2020/175473A is referred to, which is incorporated herein by reference.
As the C-terminal protective group, a condensed polycyclic aromatic hydrocarbon compound represented by Formula (1) in WO2020/175472A is also preferable. For such a compound, WO2020/175472A is referred to, which is incorporated herein by reference.
The C-terminal protective group may be a compound disclosed in WO2020/262259A (JP2019-122492 and a patent application based on the same), and WO2020/262259A (JP2019-122492 and a patent application based on the same) is referred to, which is incorporated herein by reference.
The amino acid or peptide used in the above-described C-terminal protecting step is not particularly limited, but is preferably an N-terminal protected amino acid or an N-terminal protected peptide, and more preferably an Fmoc-protected amino acid or an Fmoc-protected peptide.
In addition, it is preferable that a hydroxy group, an amino group, a carbonyl group, a carboxyl group, an amide group, an imidazole group, an indole group, a guanidyl group, a mercapto group, or the like, which is a moiety other than the C-terminal end in the amino acid or peptide used in the above-described C-terminal protecting step, is protected by a protective group.
In a case where the bonding site of the C-terminal protective group is -OH or -SH, it is preferable to add a condensing agent in the presence of a condensation additive (condensation activating agent) in a solvent which does not affect the reaction, or to react in an acid catalyst.
In a case where the bonding site of the C-terminal protective group is $-NHR^{18}$ ($R^{18}$ is a hydrogen atom, an alkyl group, an arylalkyl group, or a heteroarylalkyl group), it is preferable to add a condensing agent in the presence of a condensation additive, or to react the condensing agent with a base. As the condensation activating agent, condensing agent, and acid catalyst, WO2020/175473A and WO2020/175472A are referred to, which are incorporated herein by

reference.

As the condensing agent, a condensing agent generally used in peptide synthesis can be used without limitation in the present disclosure. Examples thereof include 4-(4,6-dimethoxy- 1,3,5-triazin-2-yl)-4-methylmorpholinium chloride (DMT-MM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3-tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3-tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC/HCl) of 1-ethyl-3-(3-dimethylaminopropyl) carbodiimide, and (benzotriazol-1-yloxy)tripyrrolidinophosphonium hexafluorophosphate (PyBoP), but the condensing agent is not limited thereto.

Among these, DIC, EDC, EDC/HCl, DMT-MM, HBTU, HATU, or COMU is preferable.

An amount of the condensing agent to be used is preferably 1 molar equivalent to 10 molar equivalent and more preferably 1 molar equivalent to 5 molar equivalent with respect to 1 molar equivalent of the substrate.

As the acid catalyst used in the condensation reaction, an acid catalyst generally used in peptide synthesis can be used without limitation, and examples thereof include methanesulfonic acid, trifluoromethanesulfonic acid, and p-toluenesulfonic acid.

Among these, methanesulfonic acid or p-toluenesulfonic acid is preferable.

An amount of the acid catalyst to be used is preferably 1 molar equivalent to 10 molar equivalent and more preferably 0.01 molar equivalent to 5 molar equivalent with respect to 1 molar equivalent of the substrate.

In the above-described C-terminal protecting step, it is preferable to add the condensation activating agent in order to promote the reaction and suppress side reactions such as racemization.

The condensation activating agent is a reagent which, in a case of coexisting with the condensing agent, leads an amino acid to a corresponding active ester, symmetric acid anhydride, or the like to facilitate the formation of a peptide bond (amide bond).

As the condensation activating agent, an activating agent generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl(hydroxyimino)cyanoacetylate (Oxyma). Among these, 4-dimethylaminopyridine, HOBt, HOCt, HOAt, HOOBt, HOSu, HONb, or Oxyma is preferable.

An amount of the activating agent to be used is preferably more than 0 molar equivalent and 4.0 molar equivalent or less and more preferably 0.1 molar equivalent to 1.5 molar equivalent with respect to 1 molar equivalent of the substrate.

As the solvent, the above-described solvent used in the above-described dissolving step can be suitably used.

The reaction temperature is not particularly limited, but is preferably -10°C to 80°C and more preferably 0°C to 40°C. The reaction time is not particularly limited, but is preferably 1 hour to 30 hours.

In addition, the N-terminal and C-terminal protected amino acid or N-terminal and C-terminal protected peptide obtained by the above-described C-terminal protecting step may be purified.

For example, in order to isolate the product obtained after dissolving the obtained N-terminal and C-terminal protected amino acid or N-terminal and C-terminal protected peptide in a solvent (reaction solvent, for example, THF) and performing a desired organic synthesis reaction. Thereafter, the solvent is changed to a solvent in which the N-terminal and C-terminal protected amino acid or N-terminal and C-terminal protected peptide is dissolved (for example, change of solvent composition or change of solvent type) to reprecipitate the resultant.

Specifically, the reaction is performed under conditions such that the N-terminal and C-terminal protected amino acid or N-terminal and C-terminal protected peptide is dissolved. After the reaction, the solvent is distilled off and then replaced, or after the reaction, by adding a polar solvent to the reaction system without distilling off the solvent, aggregates are precipitated and impurities are eliminated.

As the solvent for replacement or the polar solvent, polar organic solvents such as methanol, acetonitrile, and water are used alone or in combination. That is, the reaction is performed under conditions such that the N-terminal and C-terminal protected amino acid or N-terminal and C-terminal protected peptide is dissolved, and in the solvent replacement after the reaction, for example, a halogenated solvent, THF, or the like is used for dissolution, and a polar organic solvent such as methanol, acetonitrile, and water is used for precipitation.

<N-terminal deprotecting step>

The method for producing a peptide according to the embodiment of the present disclosure preferably includes an N-terminal deprotecting step of deprotecting an N-terminal protective group of the N-terminal and C-terminal protected amino acid or N-terminal and C-terminal protected peptide obtained in the above-described C-terminal protecting step.

As the N-terminal protective group, a protective group for an amino group described later, which is generally used in a technical field such as peptide chemistry, can be used. In the present disclosure, a Boc group, a Cbz group, or an Fmoc group is suitable.

The deprotection conditions are appropriately selected depending on the type of the temporary protective group. For example, in a case of the Fmoc group, the deprotection is performed by treating with a base, and in a case of the Boc group, the deprotection is performed by treating with an acid. The reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

As the solvent, the above-described solvent used in the above-described dissolving step can be suitably used.

<Peptide chain extending step>

The method for producing a peptide compound according to the embodiment of the present disclosure preferably includes a peptide chain extending step of condensing an N-terminal protected amino acid or an N-terminal protected peptide to the N-terminal of the C-terminal protected amino acid or C-terminal protected peptide obtained in the above-described N-terminal deprotecting step. The N-terminal protected amino acid is preferably, for example, the polycyclic compound represented by Formula (1), in which $Y^1$ is -NHCOR$^{18}$ and $R^{18}$ is the chemical structure having a protected or unprotected amino group and a carboxy group.

The above-described peptide chain extending step is preferably performed under peptide synthesis conditions generally used in the field of peptide chemistry, in which the above-described condensing agent, condensation additive, and the like are used.

The N-terminal protected amino acid or the N-terminal protected peptide is not particularly limited, and an Fmoc-protected amino acid or an Fmoc-protected peptide is suitable.

<Precipitating step>

It is preferable that the method for producing a peptide according to the embodiment of the present disclosure further includes a precipitating step of precipitating the N-terminal and C-terminal protected peptide obtained in the above-described peptide chain extending step.

The precipitating step can be performed in the same manner as in the purification (reprecipitation) of the C-terminal protecting step described above.

Specifically, a polar solvent is added to the reaction system without distilling off the reaction solvent after the reaction in the previous stage. In this case, in the reaction solvent, THF is used as the nonpolar organic solvent and acetonitrile is used as the polar solvent. The proportion (volume basis) of the nonpolar organic solvent and the polar solvent used is preferably 1:1 to 1:100, more preferably 1:3 to 1:50, and still more preferably 1:5 to 1:20. In the case of this proportion used, the N-terminal and C-terminal protected amino acid or N-terminal and C-terminal protected peptide can be efficiently precipitated, and the target product can be efficiently purified.

<Chain extension>

It is preferable that the method for producing a peptide according to the embodiment of the present disclosure further includes, one or more times in the following order after the above-described precipitating step, a step of deprotecting the N-terminal of the obtained N-terminal and C-terminal protected peptide, a step of condensing the N-terminal of the obtained C-terminal protected peptide with an N-terminal protected amino acid or an N-terminal protected peptide, and a step of precipitating the obtained N-terminal and C-terminal protected peptide.

By repeating the above-described three steps, the chain extension of the obtained peptide can be easily performed.

Each step in the above-described three steps can be performed in the same manner as each corresponding step described above.

<C-terminal deprotecting step>

It is preferable that the method for producing a peptide according to the embodiment of the present disclosure further includes a C-terminal deprotecting step of deprotecting a C-terminal protective group.

In the above-described C-terminal deprotecting step, by removing the above-described C-terminal protective group in the C-terminal protected peptide having a desired number of amino acid residues, a peptide as a final target

product can be obtained.

Preferred examples of a method of removing the C-terminal protective group include a deprotecting method using an acidic compound.

Examples thereof include a method of adding an acid catalyst and a hydrogenating method using a metal catalyst. Examples of the acid catalyst include trifluoroacetic acid (TFA), hydrochloric acid, trifluoroethanol (TFE), hexafluoroisopropanol (HFIP), and acetic acid. TFA is preferable for peptides which do not decompose with strong acids, and TFE, HFIP, or acetic acid is preferable for peptides which decompose with strong acids. A concentration of the acid can be appropriately selected depending on the side chain protective group of the extending amino acid and the deprotection conditions.

For example, a concentration of TFA is preferably 50% by volume or less, more preferably 30% by volume or less, still more preferably 10% by volume or less, even more preferably 5% by volume or less, and particularly preferably 1% by volume or less with respect to the total volume of the solvent used. The lower limit value thereof is preferably 0.01% by volume, more preferably 0.1% by volume, and still more preferably 0.5% by volume.

The deprotection time is preferably 5 hours or less, more preferably 3 hours or less, and still more preferably 1 hour or less.

In the present disclosure, the C-terminal protective group and the side chain protective group can be deprotected even under weak acid conditions, and a side reaction of the obtained peptide can be suppressed.

Examples of peptide which is suitable for deprotection of the C-terminal protective group under weak acid conditions (that is, peptide which is sensitive to acid) include peptides having an N-alkylamide structure.

From the viewpoint of suppressing side reactions of the obtained peptide and of temporal stability, the method for producing a peptide according to the embodiment of the present disclosure is preferably applied to a method for producing a peptide which is sensitive to acid, more preferably applied to a method for producing a peptide having an N-alkylamide structure.

The peptide, which is the final target product obtained by the method for producing a peptide according to the embodiment of the present disclosure, can be isolated and purified according to a method commonly used in peptide chemistry. For example, the peptide, which is the final target product, can be isolated and purified by extraction and washing the reaction mixture, crystallization, chromatography, and the like.

The type of peptide produced by the method for producing a peptide according to the embodiment of the present disclosure is not particularly limited, but it is preferable that the number of amino acid residues of the peptide is, for example, approximately several tens or less. Same as existing or unknown synthetic or native peptides, the peptide obtained by the method for producing a peptide according to the embodiment of the present disclosure can be used in various fields such as pharmaceuticals, foods, cosmetics, electronic materials, biosensors, and the like, but the use of the peptide is not limited thereto.

In the method for producing a peptide according to the embodiment of the present disclosure, the precipitating step can be appropriately omitted as long as it does not affect the reaction in the next step.

## Examples

Hereinafter, the embodiments of the present invention will be more specifically described with reference to Examples. The materials, amounts to be used, proportions, treatment contents, treatment procedures, and the like shown in Examples can be appropriately modified as long as the modifications do not depart from the spirit of the embodiments of the present invention. Therefore, the scope of the embodiments of the present invention is not limited to the following specific examples. In addition, "parts" and "%" are on a mass basis unless otherwise specified. The room temperature means 25°C.

Unless otherwise specified, purification by column chromatography was performed using an automatic purification device ISOLERA (manufactured by Biotage Ltd.) or a medium pressure liquid chromatograph YFLC-Wprep 2XYN (manufactured by YAMAZEN).

Unless otherwise specified, SNAPKP-SII Cartridge (manufactured by Biotage Ltd.) or a high flash column W001, W002, W003, W004, or W005 (manufactured by YAMAZEN) was used as a carrier in silica gel column chromatography.

The mixing ratio in an eluent used for column chromatography is the volume ratio. For example, "gradient elution of hexane:ethyl acetate = 50:50 to 0:100" means that an eluent of 50% hexane and 50% ethyl acetate is finally changed to an eluent of 0% hexane and 100% ethyl acetate.

In addition, "gradient elution of hexane:ethyl acetate = 50:50 to 0:100 and gradient elution of methanol:ethyl acetate = 0:100 to 20:80" means that an eluent of 50% hexane and 50% ethyl acetate is changed to an eluent of 0% hexane and 100% ethyl acetate, and then the eluent of 0% hexane and 100% ethyl acetate is finally changed to an eluent of 20% methanol and 80% ethyl acetate.

MS spectrum was measured using ACQUITY SQD LC/MS System (manufactured by Waters Corporation; ionization method; electrospray ionization (ESI) method).

NMR spectrum was measured using Bruker AV300 (manufactured by Bruker, 300 MHz) or Bruker AV400 (manufactured by Bruker, 400 MHz), using tetramethylsilane as an internal reference, and all δ values were represented in ppm.

The HPLC purity was measured using ACQUITY UPLC (manufactured by Waters Corporation, column: CSH C18 1.7 μm).

<Synthesis of protective group-forming reagent (synthesis of intermediate 2 and Example Compound 1)>

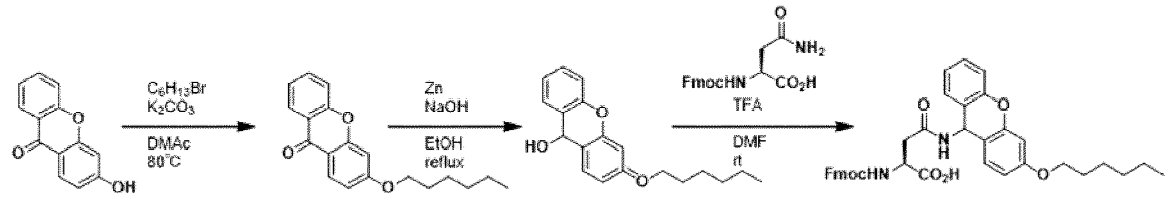


Intermediate 1 Intermediate 2 Example Compound 1

<Synthesis of intermediate 1>

Potassium carbonate (26.1 g, 188.5 mmol) and 400 ml of N,N-dimethylacetamide were added to 3-hydroxyxanthone (20.0 g, 94.3 mmol), 1-bromohexane (20.2 g, 122.5 mmol) was added dropwise thereto, and the reaction solution was heated to an internal temperature of 80°C and stirred for 2 hours. The reaction solution was cooled, 800 ml of water was added thereto, and the mixture was stirred for 30 minutes and then filtered. 1200 ml of methanol was added to the obtained crude product, the mixture was heated and refluxed to dissolve the crude product, 2400 ml of water was added thereto, and then the mixture was filtered and washed with 400 ml of water to obtain 27.4 g of an intermediate 1 as a white solid.

$^1$H-NMR (DMSOd$_6$, 400 MHz): δ = 0.88 (3H, t), 1.27 to 1.48 (6H, m), 1.73 to 1.81 (2H, m), 4.15 (2H, t), 7.05 (1H, dd), 7.15 (1H, d), 7.47 (1H, dt), 7.63 (1H, d), 7.85 (1H, dt), 8.10 (1H, d), 8.17 (1H, dd)

<Synthesis of intermediate 2>

The intermediate 1 (2.00 g, 6.75 mmol), sodium hydroxide (2.16 g, 53.99 mmol), zinc powder (0.88 g, 13.50 mmol), and 12 ml of ethanol were mixed with each other, and the mixture was stirred for 2 hours while being heated and refluxed. After allowing to cool, 28 ml of ethanol was added thereto, the mixture was filtered and washed with 20 ml of ethanol, and the filtrate was concentrated under reduced pressure using an evaporator. 20 ml of saturated saline, ammonium chloride (4.33 g, 80.98 mmol), and 20 ml of water were added to the obtained oily substance, and the mixture was further extracted with 20 ml of ethyl acetate, washed with saturated saline, dried over magnesium sulfate, filtered, and concentrated to obtain an intermediate 2. The intermediate 2 acts as a protective group-forming reagent.

<Synthesis method of Example Compound 1>

The intermediate 2 (2.02 g, 6.77 mmol), Fmoc-L-asparagine (1.43 g, 4.02 mmol), and 24 ml of dimethylformamide were mixed with each other, and trifluoroacetic acid (4.37 ml, 40.22 mmol) was added dropwise thereto and stirred for 2 hours. The reaction solution was added to 72 ml of water, the mixture was stirred for 30 minutes and then filtered, and the obtained crude product was reslurried with methanol to obtain 1.93 g (2 steps, 43.5%) of a target product.

$^1$H-NMR (DMSOd$_6$, 400 MHz): δ = 0.87 (3H, m), 1.23 to 1.38 (6H, m), 1.63 to 1.69 (2H, m), 2.55 (1H, dd), 2.68 (1H, dd), 3.89 to 3.96 (2H, m), 4.20 to 4.29 (3H, m), 4.49 (1H, q), 6.22 (1H, d), 6.61 to 6.68 (2H, m), 7.05 to 7.13 (2H, m), 7.22 to 7.44 (7H, m), 7.66 to 7.74 (3H, m), 7.91 (2H, d), 8.83 (1H, d), 12.8 (1H, br)

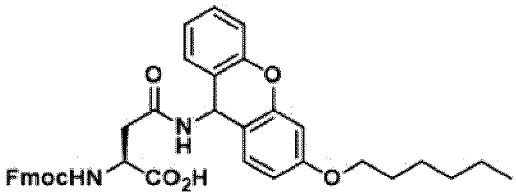

Example Compound 1

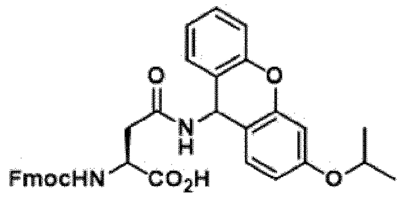

Example Compound 2

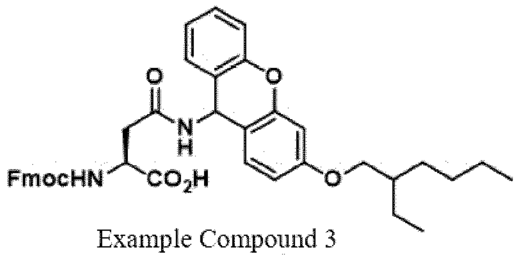

Example Compound 3

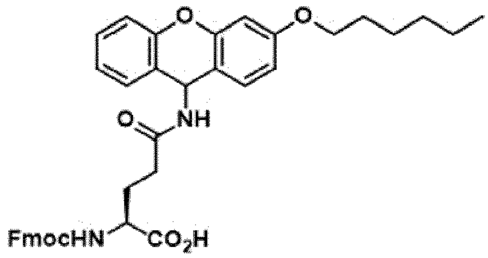

Example Compound 4

<Synthesis method of Example Compound 2>

Example Compound 2 was obtained in the same manner as in Example Compound 1, except that 1-bromohexane was changed to 2-bromopropane.

$^1$H-NMR (DMSOd$_6$, 400 MHz): $\delta$ = 1.23 (6H, m), 2.56 (1H, dd), 2.69 (1H, dd), 4.20 to 4.30 (3H, m), 4.46 to 4.63 (2H, m), 6.22 (1H, d), 6.58 to 6.68 (2H, m), 7.04 to 7.13 (2H, m), 7.23 to 7.44 (7H, m), 7.67 to 7.75 (3H, m), 7.91 (2H, d), 8.83 (1H, d), 12.8 (1H, br)

<Synthesis method of Example Compound 3>

Example Compound 3 was obtained in the same manner as in Example Compound 1, except that 1-bromohexane was changed to 2-ethylhexyl bromide.

<Synthesis method of Example Compound 4>

Example Compound 4 was obtained in the same manner as in Example Compound 1, except that Fmoc-L-asparagine was changed to Fmoc-L-glutamine.

<Comparative Compounds 1 and 3>

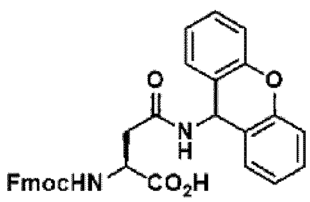

Comparative Compound 1

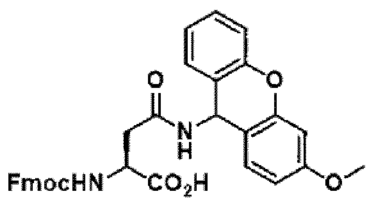

Comparative Compound 2

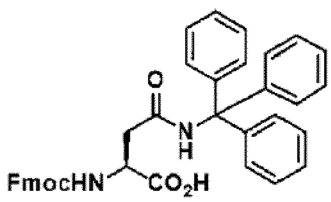

Comparative Compound 3

As Comparative Compounds 1 and 3, commercially available products manufactured by WATANABE CHEMICAL INDUSTRIES, LTD. were used.

<Synthesis method of Comparative Compound 2>

Comparative Compound 2 was synthesized in the same manner as in Example Compound 1, except that 1-bromohexane was changed to methyl p-toluenesulfonate.

(Evaluation 1)

<Deprotection rate>

With regard to the protected amino acid synthesized above, a deprotection ratio of a protected side chain site was determined as follows.

50 mg of the side chain-protected amino acid using the compound of Examples or the side chain-protected amino acid using the compound of Comparative Examples and Fmoc-Gly-OH (internal standard) in an equimolar amount of the side chain-protected amino acid were mixed, and then dichloromethane/trifluoroethanol/trifluoroacetic acid (100/10/1: vol ratio) was added thereto so that the substrate concentration was 0.025 M based on the side chain-protected amino acid, and the mixture was stirred at 30°C for 60 minutes.

5 μL of the reaction solution was dissolved in 400 μL of methanol (MeOH), and using Ultra Performance LC (ultra-performance liquid chromatography, manufactured by Waters Corporation, model number: ACQUITY), the deprotection ratio (%) was determined by quantifying the ratio of Fmoc-amino acid and Fmoc-Gly-OH produced by deprotecting the side chain-protected amino acid, and evaluated based on the following standard.

The columns and measurement conditions used for the ultra-performance liquid chromatography are shown below.

Column: manufactured by Waters Corporation, model number: BEH C18 1.7 μm, 2.1 mm × 30 mm
Flow rate: 0.5 mL/min
Solvent: solution A: 0.1% formic acid-water, solution B: 0.1% formic acid-acetonitrile
Gradient cycle: 0.00 min (solution A/solution B = 95/5), 2.00 min (solution A/solution B = 5/95), 3.00 min (solution A/solution B = 95/5)
Detection wavelength: 254 nm

Regarding the evaluation of the deprotection rate, a case of "B" or higher was regarded as acceptable. The results are shown in Table 1.

It can be said that, as the deprotection ratio is higher, the deprotection rate is higher, which is suitable.

-Evaluation standard-

"A": deprotection ratio was 90% or more.
"B": deprotection ratio was 50% or more and less than 90%.
"C": deprotection ratio was 10% or more and less than 50%.
"D": deprotection ratio was less than 10%.

<Reaction rate in condensation>

1.87 ml of tetrahydrofuran was added to benzylamine (20 mg, 0.187 mmol) and N-methylmorpholine (0.082 ml, 0.747 mmol), and the side chain-protected amino acid obtained above was further added thereto in an amount of 1.25 molar equivalent with respect to the benzylamine. Thereafter, (1-cyano-2-ethoxy-2-oxoethylidenaminooxy)dimethylamino-morpholino-carbenium hexafluorophosphate (COMU, 1.25 molar equivalent) was added thereto and stirred for 1 hour, and evaluation was performed based on the following standard. The results are shown in Table 1.

Regarding the evaluation of the reaction rate in the condensation, a case of "B" or higher with a survival rate of benzylamine as an indicator was regarded as acceptable.

As the survival rate of benzylamine is lower, the reactivity in the condensation of the side chain-protected amino acid is higher, which is suitable.

-Evaluation standard-

"A": survival rate was less than 5%.
"B": survival rate was 5% or more and less than 20%.

"C": survival rate was 20% or more and less than 50%.
"D": survival rate was 50% or more.

[Table 1]

| | Compound | Deprotection Rate | Condensation Reaction Rate |
|---|---|---|---|
| Example 1 | Example Compound 1 | A | A |
| Example 2 | Example Compound 2 | A | B |
| Example 3 | Example Compound 3 | A | A |
| Example 4 | Example Compound 4 | A | A |
| Comparative Example 1 | Comparative Compound 1 | D | D |
| Comparative Example 2 | Comparative Compound 2 | C | C |
| Comparative Example 3 | Comparative Compound 3 | C | A |

From Table 1, the compounds used in Examples 1 to 4 were superior in both deprotection rate and reaction rate in the condensation as compared with the compound of Comparative Examples 1 to 3.

<Synthesis of protected peptide (4-residue peptide: Fmoc-Phe-Gln(X)-Asn(X)-Arg-Cys(Trt)-OH)>

Details of each abbreviation other than the above are shown below.

Phe: phenylalanine residue
Gln(X): glutamine residue having the protective group according to the present invention
Asn(X): asparagine residue having the protective group according to the present invention
Cys(Trt): Trt-protected cysteine residue
Trt: triphenylmethyl group

(Example 5: synthesis of Fmoc-Cys(Trt)-O-NaphTAG)

A raw material 1 (914 mg, 1.00 mmol) synthesized according to Examples of WO2020/175472A, Fmoc-Cys(Trt)-OH (879 mg, 1.50 mmol), and tetrahydrofuran (10 mL) were mixed with each other at room temperature, and 4-dimethylaminopyridine (24.4 mg, 0.20 mmol) and diisopropylcarbodiimide (232 μL, 1.50 mmol) were added thereto. After stirring the reaction solution for 1 hour under nitrogen, methanol (50 mL) was added thereto to precipitate solid, and the solid was filtered and dried under reduced pressure to obtain Fmoc-Cys(Trt)-O-NaphTAG (1.452 g, 98.0%).

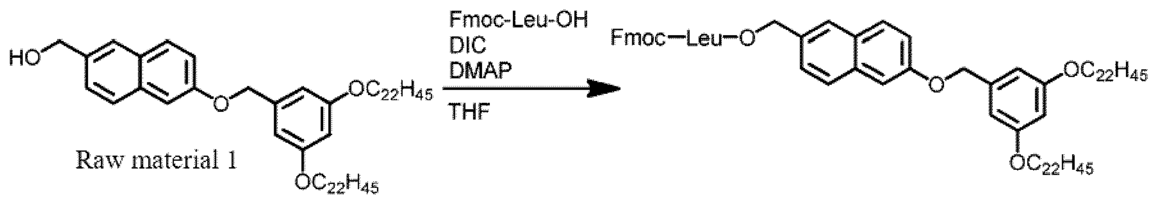


(Example 6: synthesis of Fmoc-Asn(Xi)-Cys(Trt)-O-NaphTAG)

Fmoc-Cys(Trt)-O-NaphTAG (1.442 g, 0.974 mmol) was dissolved in tetrahydrofuran (9.7 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Example Compound 1 (denoted as Asn(Xi)) (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (48.7 mL) was added thereto and stirred, and then the precipitate was filtered, washed with acetonitrile, and dried under reduced pressure to obtain Fmoc-Asn($X_1$)-Cys(Trt)-O-NaphTAG (1.775 g, yield: 97.2%).

(Example 7: synthesis of Fmoc-Gln(Y)-Asn(Xi)-Cys(Trt)-O-NaphTAG)

Fmoc-Asn(Xi)-Cys(Trt)-O-NaphTAG (1.765 g, 0.941 mmol) was dissolved in tetrahydrofuran (9.4 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Example Compound 4 (denoted as Gln(Y)) (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (47 mL) was added thereto and stirred, and then the precipitate was filtered, washed with acetonitrile, and dried under reduced pressure to obtain Fmoc-Gln(Y)-Asn(Xi)-Cys(Trt)-O-NaphTAG (2.100 g, yield: 97.7%).

$$\text{ESI-MS(+)} = 1985.1$$

(Example 8: synthesis of Fmoc-Phe-Gln(Y)-Asn(Xi)-Cys(Trt)-O-NaphTAG)

Fmoc-Gln(Y)-Asn(Xi)-Cys(Trt)-O-NaphTAG (2.09 g, 0.915 mmol) was dissolved in tetrahydrofuran (9.2 mL), and diazabicycloundecene (DBU, 2.0 molar equivalent) was added thereto and stirred. After the deprotection reaction was completed, N-methylmorpholine (2.05 molar equivalent) and methanesulfonic acid (2.0 molar equivalent) were added thereto in sequence, and Fmoc-Phe-OH (1.25 molar equivalent) and (1-cyano-2-ethoxy-2-oxoethylidenaminooxy)dimethylamino-morpholino-carbenium hexafluorophosphate (COMU, 1.25 molar equivalent) were added thereto and stirred. After the condensation reaction was completed, acetonitrile (46 mL) was added thereto and stirred, and then the precipitate was filtered, washed with acetonitrile, and dried under reduced pressure to obtain Fmoc-Phe-Gln(Y)-Asn(Xi)-Cys(Trt)-O-NaphTAG (2.167 g, yield: 97.4%).

(Example 9: deprotection of peptide)

At room temperature, trifluoroacetic acid/hexafluoroisopropanol/dichloromethane (1/10/100: vol%, 88.7 mL), triisopropylsilane (10.0 molar equivalent), and 3,6-dioxa-1,8-octanedithiol (10.0 molar equivalent) were added to Fmoc-Phe-Gln(Y)-Asn(Xi)-Cys(Trt)-O-NaphTAG (2.157 g, 0.887 mmol) and stirred for 1 hour. A 1/1 mixed solution (1 L) of Tert-butylmethyl ether and normal hexane was added to the reaction solution for crystallization to obtain Fmoc-Phe-Gln-Asn-Cys-OH (586 mg, 90.2%).

HPLC purity (220 nm): 88.5%
MS (ESI, m/Z): 733.8 (M + H)

(Examples 10 to 13)

Peptides were synthesized in the same manner as in Examples 6 to 9, except that the asparagine compound was changed from Example Compound 1 to Example Compound 2 (denoted as Asn($X_2$)).

(Example 13)

HPLC purity (220 nm): 86.0%

[Table 2]

| | Sequence | Yield [%] |
|---|---|---|
| Example 5 | Fmoc-Cys(Trt)-O-NaphTAG | 98.0 |
| Example 6 | Fmoc-Asn($X_1$)-Cys(Trt)-O-TAG | 97.2 |
| Example 7 | Fmoc-Gln(Y)-Asn($X_1$)-Cys(Trt)-O-TAG | 97.7 |
| Example 8 | Fmoc-Phe-Gln(Y)-Asn($X_1$)-Cys(Trt)-O-TAG | 97.4 |
| Example 9 | Fmoc-Phe-Gln-Asn-Cys-OH | 90.2 |
| Example 10 | Fmoc-Asn($X_2$)-Cys(Trt)-O-TAG | 96.8 |
| Example 11 | Fmoc-Gln(Y)-Asn($X_2$)-Cys(Trt)-O-TAG | 97.0 |

(continued)

| | Sequence | Yield [%] |
|---|---|---|
| Example 12 | Fmoc-Phe-Gln(Y)-Asn($X_2$)-Cys(Trt)-O-TAG | 96.5 |
| Example 13 | Fmoc-Phe-Gln-Asn-Cys-OH | 91.5 |

As shown in Examples, with the method for producing a peptide according to the embodiment of the present disclosure, the peptide could be synthesized in high yield, the side chain-protective group could be deprotected even under weak acid conditions, and the purity was high.

## Claims

1. A method for producing a peptide, comprising:

   a step of using a polycyclic compound represented by Formula (1),

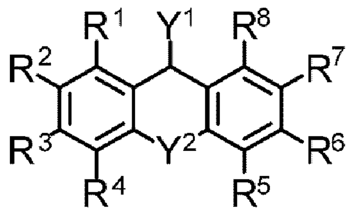

(1)

   in Formula (1), $Y^1$ represents $-OR^{17}$, $-NHR^{18}$, $-NHCOR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group, an arylalkyl group, a heteroarylalkyl group, or a chemical structure having a protected or unprotected amino group and a carboxy group,
   $Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$ each independently represent a hydrogen atom or an alkyl group, and $R^{110}$ represents $R^A$ or a hydrogen atom,
   $R^1$ to $R^8$ each independently represent $R^A$, a hydrogen atom, a halogen atom, an alkyl group, or an alkoxy group, where at least one of $R^1$ to $R^8$ and $R^{110}$ is $R^A$, and
   $R^A$'s each independently represent an aliphatic hydrocarbon group or an organic group having an aliphatic hydrocarbon group, where at least one aliphatic hydrocarbon group has 3 or more carbon atoms.

2. The method for producing a peptide according to claim 1,
   wherein the step of using the compound represented by Formula (1) is a side chain protecting step of protecting a protected or unprotected side chain amino group of asparagine or glutamine with the compound represented by Formula (1).

3. The method for producing a peptide according to claim 1,

   wherein the compound represented by Formula (1) is an N-terminal protected amino acid or an N-terminal protected peptide, and
   the method for producing a peptide further comprising:

   a peptide chain extending step of condensing the compound with a C-terminal protected amino acid or a C-terminal protected peptide; and
   a precipitating step of precipitating an N-terminal and C-terminal protected peptide obtained in the peptide chain extending step.

4. The method for producing a peptide according to claim 3, further comprising, one or more times in the following order after the precipitating step:

   a step of deprotecting an N-terminal of the obtained N-terminal and C-terminal protected peptide;

a step of condensing an N-terminal of the obtained C-terminal protected peptide with an N-terminal protected amino acid or an N-terminal protected peptide; and
a step of precipitating the obtained N-terminal and C-terminal protected peptide.

**5.** The method for producing a peptide according to claim 3 or 4, further comprising:

a C-terminal deprotecting step of deprotecting a C-terminal protective group,
wherein the deprotecting is performed using a trifluoroacetic acid solution of 10% by volume or less.

**6.** The method for producing a peptide according to any one of claims 3 to 5,
wherein a C-terminal protective group of the C-terminal protected amino acid or the C-terminal protected peptide has an aliphatic hydrocarbon group having 12 or more carbon atoms.

**7.** The method for producing a peptide according to any one of claims 1 to 6,
wherein only one of $R^1$ to $R^8$ in Formula (1) is $R^A$.

**8.** The method for producing a peptide according to any one of claims 1 to 7,
wherein $R^3$ or $R^6$ in Formula (1) is $R^A$.

**9.** The method for producing a peptide according to any one of claims 1 to 8,
wherein $R^A$'s in Formula (1) are each independently an alkyl group having 3 to 10 carbon atoms, an alkoxy group having 3 to 10 carbon atoms, or an alkoxyalkyl group having 3 to 10 carbon atoms.

**10.** The method for producing a peptide according to any one of claims 1 to 7,
wherein $Y^2$ in Formula (1) is -O-.

**11.** A protective group-forming reagent of a side chain amino of arginine or glutamine, comprising:

a polycyclic compound represented by Formula (1),

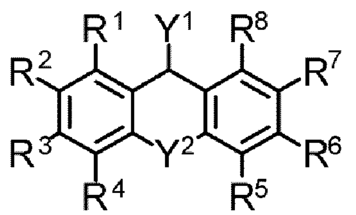

(1)

in Formula (1), $Y^1$ represents $-OR^{17}$, $-NHR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group, an arylalkyl group, or a heteroarylalkyl group,
$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$ each independently represent a hydrogen atom or an alkyl group, and $R^{110}$ represents $R^A$ or a hydrogen atom,
$R^1$ to $R^8$ each independently represent $R^A$, a hydrogen atom, a halogen atom, an alkyl group, or an alkoxy group, where at least one of $R^1$ to $R^8$ and $R^{110}$ is $R^A$, and
$R^A$ represents an aliphatic hydrocarbon group or an organic group having an aliphatic hydrocarbon group, where at least one aliphatic hydrocarbon group has 3 to 10 carbon atoms.

**12.** A polycyclic compound represented by Formula (1),

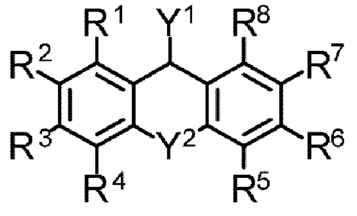

(1)

in Formula (1), $Y^1$ represents $-OR^{17}$, $-NHR^{18}$, $-NHCOR^{18}$, -SH, or a halogen atom, where $R^{17}$ represents a hydrogen atom, an active ester-type carbonyl group, or an active ester-type sulfonyl group, and $R^{18}$ represents a hydrogen atom, an alkyl group having 10 or less carbon atoms, an arylalkyl group, a heteroarylalkyl group, or a chemical structure having a protected or unprotected amino group and a carboxy group,
$Y^2$ represents -O-, -S-, $-CR^{100}=CR^{101}-$, $-CR^{102}R^{103}-CR^{104}R^{105}-$, $-CR^{106}R^{107}-$, or $-N(R^{110})-$, where $R^{100}$ to $R^{107}$ each independently represent a hydrogen atom or an alkyl group, and $R^{110}$ represents $R^A$ or a hydrogen atom,
$R^1$ to $R^8$ each independently represent $R^A$, a hydrogen atom, a halogen atom, an alkyl group having 1 to 4 carbon atoms, or an alkoxy group having 1 to 4 carbon atoms,
at least one of $R^2$ to $R^7$ and $R^{110}$ is $R^A$, and
$R^A$ represents an aliphatic hydrocarbon group or an organic group having an aliphatic hydrocarbon group, where at least one aliphatic hydrocarbon group has 3 to 10 carbon atoms.

**13.** The polycyclic compound according to claim 12,

wherein the polycyclic compound is represented by Formula (2),

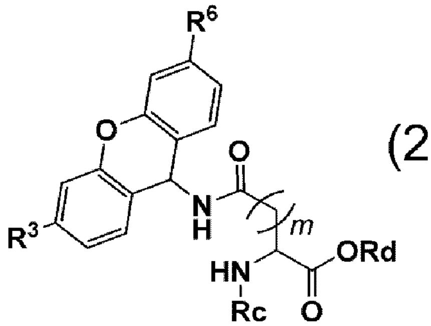

(2)

in Formula (2), $R^3$ and $R^6$ each independently represent a hydrogen atom or an alkoxy group having 3 to 10 carbon atoms, where at least one of $R^3$ or $R^6$ is an alkoxy group having 2 to 10 carbon atoms,
Rc represents a hydrogen atom, a tert-butoxycarbonyl group, or a 9-fluorenylmethoxycarbonyl group, Rd represents a hydrogen atom or a cation having a salt structure, and m represents 1 or 2.

**INTERNATIONAL SEARCH REPORT**

International application No.
**PCT/JP2021/047183**

**A. CLASSIFICATION OF SUBJECT MATTER**

***C07K 1/06***(2006.01)i; ***C07C 269/06***(2006.01)i; ***C07C 271/22***(2006.01)i; ***C07D 311/88***(2006.01)i
FI: C07K1/06 ZNA; C07C269/06; C07D311/88; C07C271/22 CSP

According to International Patent Classification (IPC) or to both national classification and IPC

**B. FIELDS SEARCHED**

Minimum documentation searched (classification system followed by classification symbols)

C07K1/06; C07C269/06; C07C271/22; C07D311/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2018/021233 A1 (SEKISUI MEDICAL CO., LTD.) 01 February 2018 (2018-02-01) claims 1, 12 | 1-2, 7-12 |
| Y | | 3-6 |
| A | | 13 |
| X | US 2014/0094567 A1 (ALBERICIO, F. et al.) 03 April 2014 (2014-04-03) claim 1 | 1, 7-10, 12 |
| A | | 2-6, 11, 13 |
| X | JP 06-145126 A (SHIMADZU CORP.) 24 May 1994 (1994-05-24) claim 1, paragraph [0051] | 1, 7-9, 12 |
| A | | 2-6, 10-11, 13 |
| X | WO 2019/184089 A1 (HYBIO PHARMACEUTICAL CO., LTD.) 03 October 2019 (2019-10-03) claim 1, example 2 | 1, 8, 10, 12 |
| A | | 2-7, 9, 11, 13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 January 2022** | **08 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/047183**

**C. DOCUMENTS CONSIDERED TO BE RELEVANT**

| Category* | Citation of document, with indication, where appropriate, of the relevant passages | Relevant to claim No. |
|---|---|---|
| X | 野田昌樹ほか. ジベンゾスベリル基及びジベンゾスベレニル基のペプチド固相合成におけるアミノ保護基の応用（第１尭）. 島津評論. vol. 54, no. 1, 1997, pp. 49-53, (Shimadzu Review), non-official translation (NODA, Masaki et al. Application of Amino-Protecting Groups in Dibenzosuberyl-Group and Dibenzosuberenyl-Group Peptide Solid Phase Synthesis (Part 1).)<br>abstract, fig. 3 | 1, 7-9, 12 |
| A | | 2-6, 10-11, 13 |
| X | HAN, Y. et al. Preparation and Applications of Xanthenylamide (XAL) Handles for Solid-Phase Synthesis of C-Terminal Peptide Amides under Particularly Mild Conditions. J. Org. Chem. 1996, vol. 61, no. 18, pp. 6326-6339<br>abstract, scheme 1, p. 6328, left column, second paragraph to p. 6329, right column, second paragraph | 1, 7-10, 12 |
| A | | 2-6, 11, 13 |
| Y | WO 2012/165546 A1 (AJINOMOTO CO., INC.) 06 December 2012 (2012-12-06)<br>examples 1, 2 | 3-6 |
| A | | 1-2, 7-13 |
| P, X | WO 2020/262259 A1 (FUJIFILM CORP.) 30 December 2020 (2020-12-30)<br>claim 1 | 1, 7-8, 10, 12 |
| P, A | | 2-6, 9, 11, 13 |

**INTERNATIONAL SEARCH REPORT**
**Information on patent family members**

International application No.
**PCT/JP2021/047183**

| Patent document cited in search report | Publication date (day/month/year) | Patent family member(s) | Publication date (day/month/year) |
|---|---|---|---|
| WO 2018/021233 A1 | 01 February 2018 | US 2019/0263842 A1<br>claims 1, 12<br>EP 3489245 A1<br>CN 109476684 A<br>KR 10-2019-0033516 A<br>TW 201803862 A | |
| US 2014/0094567 A1 | 03 April 2014 | US 2014/0343227 A1<br>WO 2012/055509 A1<br>EP 2632936 A1<br>TW 201249861 A<br>CA 2816175 A<br>CN 103189384 A<br>SG 190066 A<br>IL 226022 D<br>KR 10-2013-0127446 A | |
| JP 06-145126 A | 24 May 1994 | US 5442122 A<br>claim 1, columns 13, 14<br>EP 597400 A1 | |
| WO 2019/184089 A1 | 03 October 2019 | CN 110317188 A | |
| WO 2012/165546 A1 | 06 December 2012 | US 2014/0088291 A1<br>examples 1, 2<br>EP 2716650 A1 | |
| WO 2020/262259 A1 | 30 December 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018021233 A
- WO 2010113939 A
- WO 2020175473 A
- WO 2020175472 A
- WO 2020262259 A
- JP 2019122492 A


### Non-patent literature cited in the description


- Protective Groups in Organic Synthesis. John Wiley and Sons, 1980
- *Organic Process Research & Development,* 2017, vol. 21 (3), 365-369